# EUROPÄISCHE PATENTSCHRIFT

(11) **EP 1 471 894 B1**
(45) Veröffentlichungstag und Bekanntmachung des Hinweises auf die Patenterteilung: **10.10.2007**
(21) Anmeldenummer: 03702605.1
(22) Anmeldetag: 06.02.2003
(51) Int. Cl.: A61K 9/72

(54) **CYCLODEXTRINE ALS SUSPENSIONSSTABILISATOREN IN DRUCKVERFLÜSSIGTEN TREIBMITTELN**
CYCLODEXTRINES FOR USE AS SUSPENSION STABILIZERS IN PRESSURE-LIQUEFIED PROPELLANTS
CYCLODEXTRINES UTILISEES COMME STABILISATEURS DE SUSPENSION DANS DES AGENTS PROPULSEURS FLUIDISES SOUS PRESSION

(30) Priorität: 07.02.2002 DE 10205087
(43) Veröffentlichungstag der Anmeldung: 03.11.2004
(73) Patentinhaber: PHARMATECH GmbH, 24220 Flintbek (DE)
(72) Erfinder: MÜLLER, Bernd, W., D-24220 Flintbek (DE); STECKEL, Hartwig, D-24232 Schönkirchen (DE); WEHLE, Sebastian, D-24113 Kiel (DE)
(74) Vertreter: UEXKÜLL & STOLBERG
(86) Internationale Anmeldenummer: PCT/EP2003/001180
(87) Internationale Veröffentlichungsnummer: WO 2003/066031

(56) Entgegenhaltungen:
- WO-A-00/47203
- WO-A-01/34213
- WO-A-99/65460
- WILLIAMS IIIA R O ET AL: "INFLUENCE OF FORMULATION TECHNIQUE FOR HYDROXYPROPYL-BETA- CYCLODEXTRIN ON THE STABILITY OF ASPIRIN IN HFA 134A" EUROPEAN JOURNAL OF PHARMACEUTICS AND BIOPHARMACEUTICS, ELSEVIER SCIENCE PUBLISHERS B.V., AMSTERDAM, NL, Bd. 47, Nr. 2, März 1999 (1999-03), Seiten 145-152, XP000803170 ISSN: 0939-6411

## Beschreibung

Die vorliegende Erfindung betrifft ein Suspensionsdosieraerosol, das mindestens ein natives oder modifiziertes Cyclodextrin enthält, ein Verfahren zur Herstellung des Dosieraerosols sowie die Verwendung von Cyclodextrinen in einem Dosieraerosol.

Dosieraerosole sind Aerosole, die durch ein druckverflüssigtes Gas und ein Dosierventil die inhalative Anwendung von Wirkstoffen beim Menschen oder Tier ermöglichen. Dosieraersole vereinen die Vorteile einer hohen Dosiergenauigkeit, einer Unabhängigkeit des Patienten durch die Portabilität sowie einer hohen Anzahl von verfügbaren Dosen. Gegenüber der oralen Gabe von Wirkstoffen besitzen Dosieraerosole zusätzlich ein geringeres Nebenwirkungspotenial.

Gegenwärtig gibt es zwei Methoden, um die Schwierigkeiten, die bei der Formulierung eines stabilen Aerosols auftreten, zu umgehen. Die erste Herstellungsmethode besteht darin, die Wirkstoffe in dem Treibmittel unter Zuhilfenahme von einem Co-Solvens unter Bildung einer echten Lösung zu lösen. Diese Methode wurde z.B. von Steckel und Müller beschrieben (H.Steckel und B.W. Müller, Metered-dose inhaler formulations with beclomethasone dipropionate using the ozone friendly propellant R 134a, Eur. J. Pharm. Biopharm. (1997) 77-83). Durch die Zugabe von Ethanol als Co-Solvens kann der Wirkstoff, in diesem Fall das Beclometasondipropionat, in Lösung gehalten werden. Da sich die meisten Wirkstoffe jedoch auch durch den Einsatz hoher Ethanolkonzentrationen nicht in den modernen Treibmitteln wie 2H-Heptafluorpropane (HFA 227) und 1,1,1,2-Tetrafluorethan (HFA 134a) lösen, ist diese Methode nicht universell einsetzbar.

Die zweite Methode der Herstellung eines Dosieraerosols besteht in der Suspendierung des Wirkstoffes in dem Treibmittel durch die Zugabe von Hilfsstoffen. Für eine hohe Wirksamkeit des Aerosols in den Atemwegen ist die Partikelgröße der Wirkstoffkomponente von entscheidender Bedeutung. Partikel, die kleiner als 5 µm sind, gelten als lungengängig. Partikel, die kleiner als 3 µm sind, erreichen die tiefen Abschnitte der Lunge und gelangen so schnell in den Körperkreislauf oder wirken vor Ort. Die Herstellung eines Suspensionsdosieraerosols beinhaltet folglich die Herstellung und Aufrechterhaltung einer geeigneten Partikelgrößenverteilung. Die Zugabe von Hilfsstoffen kann die im Treibmittel suspendierten Partikel z.B. hinsichtlich einer Verklumpung der Partikel, einer Wandbeladung des Transportcontainers oder einer Sedimentation oder Aufrahmung stabilisieren.

Nachteile einer derartigen Herstellungsmethode sind die aufwendige Einstellung der Partikelgrößenverteilung der Wirkstoffkomponente auf eine inhalierbare Größe sowie die Vermeidung einer Änderung der Größenverteilung über einen geeigneten Lagerungszeitraum. Um den zerkleinerten Wirkstoff ausreichend im verflüssigten Treibmittel zu stabilisieren, wird ein Tensid zugesetzt, das sich im Treibmittel löst. Bisher werden die Tenside Ölsäure, Lecithin und Sorbitantrioleat verwendet. Diese drei für die inhalative Anwendung zugelassenen Tenside sind jedoch in den modernen Treibmitteln (2H-Heptafluorpropane (HFA 227) und 1,1,1,2-Tetrafluorethan (HFA 134a)) nicht in ausreichender Menge löslich, so daß keine Suspensionsstabilisierung stattfindet. Daher muß man versuchen, die Tenside durch den Einsatz eines Co-Solvens wie Ethanol in Lösung zu halten, um einen suspensionsstabilisierenden Effekt zu erreichen, wie es z.B. in der EP-B1-0 372 777 offenbart ist. In einigen wenigen Fällen mag auch eine stabile Suspension ohne weitere Zugabe von Tensiden oder anderen Hilfsstoffen resultieren, wie von Steckel und Müller (H.Steckel und B.W. Müller, Metered-dose inhaler formulation of fluticasone-17-propionate micronized with super-critical carbon dioxide using the alternative propellant HFA-227, Int. J. Pharm. 173 (1998) 25-33) und in der US-B-5 891 420 offenbart wird. Eine andere Möglichkeit besteht darin, nach neuen Tensiden zu suchen, die eine bessere Löslichkeit in den Treibmitteln besitzen (DE-A-42 08 545 und US-B-6 054 488). In den beiden letztgenannten Schriften wird beispielsweise der Einsatz von ethoxylierten und nicht-ethoxylierten Partialgly-ceriden als Suspensionsstabilisator offenbart.

Aus der WO 99/65460 ist ein Dosieraerosol bekannt, das einen β-Agonisten, mindestens ein Fluoralkantreibmittel und mehr als 5 Gew.-% eines Lösungsmittels umfaßt, das in der Lage ist, den β-Agonisten zu solubilisieren oder aufzulösen. Das Fluoralkan kann beispielsweise 1,1,1,2-Tetrafluorethan sein. Das Lösungsmittel ist beispielsweise Ethanol. Alternativ zu dem Lösungsmittel können zur Suspensierung des mikronisierten Wirkstoffs in dem Treibmittel beispielsweise Tenside vorhanden sein, die Bildung einer Suspension der Teilchen des β-Agonisten fördern. Die Formulierungen gemäß dieser Druckschrift werden hergestellt, indem Lösungsmittel oder Tensid vorgelegt werden, der feste Wirkstoff zugefügt wird, Treibmittel zugefügt wird und die Formulierung 5 Minuten lang mit Ultraschall behandelt wird.

Die genannten Methoden sind jedoch nur auf bestimmte Wirkstoffe und nicht universell anwendbar. Hinzu kommt, da die Umstellung der Treibmittel von ozonschädlichen FCKWs auf die ozonfreundlichen FKWs wie 2H-Heptafluorpropan (HFA 227) und 1,1,1,2-Tetrafluorethan (HFA 134a) durch die unterschiedlichen physikochemischen Eigenschaften der neuen Treibmittel zu Problemen bei der Herstellung von Dosieraerosolen führt.

Damit lag der Erfindung die Aufgabe zugrunde, eine Zusammensetzung zur Verfügung zu stellen, in der beliebige pharmazeutische Wirkstoffe mit den modernen Treibmitteln, insbesondere HFA 227 und HFA 134a, zu stabilen Suspensionsdosieraerosolen formuliert sind.

Aus der EP-A-0 349 091 ist bekannt, daß unter Verwendung von Dimethyl-β-Cyclodextrin pharmazeutische Zusammensetzungen, die 17-β-Östradiol und/oder Progesteron enthalten, zur nasalen Verabreichung geeignet sind. Die Veröffentlichungsschrift beschäftigt sich jedoch unter anderem nicht mit den Problemen, die mit HFA-Treibmitteln auftreten, sondern mit wäßrigen Lösungen.

Die WO 90/15792 offenbart Einschlußkomplexe von Cyclodextrinen mit bestimmten Wirkstoffen in wäßriger Lösung zur Behandlung von Herzerkrankungen, jedoch nicht Dosieraerosole.

Die DE-A-31 18 218 offenbart, daß durch Methylierung von β-Cyclodextrinen die Wasserlöslichkiet von Einschlußkomplexen der Cyclodextrine mit biologisch aktiven organischen Substanzen verbessert wird.

In Vorversuchen für die vorliegende Erfindung wurde festgestellt:
1. Eine Kombination von Cyclodextrin mit Ethanol liefert mit Treibmittel und Wirkstoff keine stabile Suspension. Es entsteht nach der Zugabe von Treibmittel zu der Mischung von Ethanol, Cyclodextrin und Wirkstoff eine milchige Suspension, die innerhalb eines Tages die typischen Nachteile einer klassischen Suspension zeigt. Die Partikel ballen sich zusammen und schwimmen auf dem Treibmittel auf. Dieses Verklumpen läßt sich auch durch intensives Schütteln nicht wieder rückgängig machen. Eine Anwendung des Dosieraerosols wird zusätzlich durch ein potentielles Versagen des Dosierventils unmöglich gemacht.
2. Eine Kombination von Polyethylenglykol mit Ethanol liefert mit Treibmittel und Wirkstoff keine stabile Suspension. Aus der Mischung von Polyethylenglykol mit Ethanol und Wirkstoff entsteht bei Zugabe von Treibmittel zuerst visuell eine Lösung. Nach Lagerung über eine Woche zeigt die Zubereitung ein deutliches Kristallwachstum des vormals gelösten Arzneistoffes. Derartige Formulierungen verlie-ren ihre therapeutische Wirksamkeit durch Entzug des Arzneistoffes und sind somit für ein exaktes Dosieren unbrauchbar.

Erfindungsgemäß wurde nun eine Suspensionsdosieraerosolzusammensetzung gefunden, die
a) mindestens einen pharmazeutischen Wirkstoff,
b) mindestens ein Treibmittel, das aus 2H-Heptafluorpropan (HFA 227), 1,1,1,2-Tetrafluorethan (HFA 134a) oder einer Mischung aus beiden ausgewählt ist,
c) mindestens ein natives oder ein modifiziertes Cyclodextrin und
d) mindestens ein hydrophiles Additiv umfaßt, das einen Polyethylenglykolanteil von 2 oder mehr Ethylenoxideinheiten besitzt,
wobei die Suspension dadurch hergestellt wird, daß ein Gemisch das a) mindestens einen pharmazeutischen Wirkstoff, c) mindestens ein natives oder ein modifiziertes Cyclodextrin, d) mindestens ein hydrophiles Additiv umfaßt, das einen Polyethylenglykolanteil von 2 oder mehr Ethylenoxideinheiten besitzt, vorgelegt wird und das Gemisch durch Zugabe von b) mindestens einem Treibmittel, das aus 2H-Heptafluorpropan (HFA 227), 1,1,1,2-Tetrafluorethan (HFA 134a) oder einer Mischung aus beiden ausgewählt ist, in eine stabile, nicht-sedimentierende Suspension überführt wird.

Diese Suspension zeigt überraschenderweise über einen längeren Lagerungszeitraum (länger als 6 Monate) optisch keine Anzeichen einer Alterung.

Die erfindungsgemäße Suspension zeigt keine Anzeichen einer Trennung in ihre Komponenten, es kommt nicht zum Aufschwimmen des Wirkstoffes auf dem Treibmittel (Flotation) oder zum Zusammenballen zu einem Kuchen auf dem Boden des Gefäßes, welches die Zubereitung enthält. Erfindungsgemäße Suspensionsdosieraerosolzusammensetzungen zeigen eine hohe inhalierbare Fraktion, bei einer hohen Dosiergenauigkeit der Formulierung und voller Funktionsfähigkeit des Dosierventils. Dabei kommt es bei Zusammengabe der genannten Komponenten zu einer spontanen Bildung einer stabilen Suspension.

Eine bevorzugte erfindungsgemäße Zusammensetzung erhält ferner e) Ethanol. Weitere bevorzugte Ausführungsformen sind Gegenstand der Unteransprüche.

Beispiele für bevorzugt eingesetzte pharmazeutische Wirkstoffe sind Antiasthmatika wie Budesonid, Beclometason, Dexamethason, Flunisolid, Fluticason, Hydrocortison, Triamcinolon, Adrenalin, Bitolterol, Clenbuterol, Ephedrin, Fenoterol, Formoterol, Isoproterenol, Noradrenalin, Pirbuterol, Reproterol, Salbutamol, Salmeterol", Terbutalin, Ipratropium, Oxitropium, Tiotropium, Nedocromil, Cromoglicinsäure, Salze oder Ester der vorstehend genannten Verbindungen oder Kombinationen der genannten Wirkstoffe; systemisch wirksame Substanzen wie Atropin, Buprenorphin, Fentanyl, Morphin, Glibenclamid, Prednison, Prednisolon, Scopolamin, Sildenafil, Apomorphin oder deren Salze und Derivate; Antiinfektiva wie z.B. Tobramycin, Gentamycin, Ciclosporin; systemisch wirksame Proteine, Peptide, Plasmide oder DNA-Fragmente wie z.B. Insulin, α₂-Antitrypsin, Calcitonin, Desmopressin, humanes Wachstumshormon und andere hormonell wirksame Sub-stanzen sowie systemische Vaccine oder Immunglobuline.

Die eingesetzten Treibmittel sind 2H-Heptafluorpropan (HFA 227), 1,1,1,2-Tetrafluorethan (HFA 134a) und Mischungen derselben.

Das erfindungsgemäß eingesetzte Cyclodextrin kann ein natives oder modifiziertes α-, β- oder γ-Cyclodextrin sein. Beispiele für modifizierte Cyclodextrine sind Hydroxymethyl-α-Cyclodextrin, Hydroxyethyl-α-Cyclodextrin, Hydroxypropyl-α-Cyclodextrin, α-Cyclodextrinbutylsulfonat, α-Cyclodextrinbutylfluorid und Sulfobutyl-α-Cyclodextrin;Hydroxymethl-β-Cyclodextrin,Hydroxyethyl-β-Cyclodextrin, Hydroxypropyl-β-Cyclodextrin, β-Cyclodextrinbutylsulfonat, β-Cyclodextrinbutylfluorid und Sulfobutyl-β-Cyclodextrin sowie Hydroxymethyl-γ-Cyclodextrin, Hydröxyethyl-γ-Cyclodextrin, Hydroxypropyl-γ-Cyclodextrin, γ-Cyclodextrinbutylsulfonat, γ-Cyclodextrinbutylfluorid und Sulfobutyl-γ-Cyclodextrin.

Das hydrophile Additiv kann gemäß bevorzugter Ausführungsformen aus der Gruppe der Polyethylenglykole mit einem mittleren Molekulargewicht von 100 bis 30000, vorzugsweise mit einem mittleren Molekulargewicht von 200 bis 6000, und bevorzugt mit einem Molekulargewicht von 200 bis 1000, ausgewählt sein. Vorzugsweise ist das hydrophile Additiv eine Mischung aus Polyethylenglykolen mit niedrigem und hohem Molekulargewicht darstellt. Insbesondere ist das hydrophile Additiv ausgewählt aus der Gruppe der Polyethylenglykol-Fettsäureether (Brij-Substanzen), der Gruppe der Polyethylenglykol-Fettsäureester (Myrj-Substanzen), der Gruppe der Polyethylenglykol-Glycerol-Fettsäureester (z.B. Tagat S, Tagat O, Solutol HS 15, Cremophor EL, Cremphor S9), der Gruppe der Polysorbate oder der Gruppe der Polyoxyethylen-polypropylen-Block-Co-Polymere, z.B. Poloxamer 188, Synperonic P/E L 61, Pluronic F 68. Besonders bevorzugt ist das hydrophile Additiv ein hydrophiles Polymer, insbesondere Polyvinylpyrrolidon, Polyvinylalkohol, quervernetztes Polyvinylpyrrolidon oder ein Vinylpyrrolidon-Vinylacetat-Copolymer.

In einer bevorzugten Ausführungsform beträgt der Gesamtgehalt der Komponenten c) und d) sowie gegebenenfalls e) 0,01 bis 30 Gew.-%, bevorzugt 0,1 bis 20 Gew.-%, insbesondere 0,5 bis 15 Gew.-%, bezogen auf die Gesamtmasse der Zubereitung.

Bevorzugt liegt das Gewichtsverhältnis' Komponente a) Wirkstoff (e) zu Komponente c) Cyclodextrin(e) im Bereich von 10 zu 1 bis 1 zu 100, bevorzugt 5 zu 1 bis 1 zu 50, insbesondere 2 zu 1 bis 1 zu 20. In bevorzugten erfindungsgemäßen Zusammensetzungen macht die Treibmittelkomponente b) 50 bis 99 Gew.-% aus, bevorzugt 75 bis 98 Gew.-%, insbesondere 80 bis 97 Gew.-%, zum Beispiel 90 bis 95 Gew.-%, bezogen auf die Gesamtmasse der Zubereitung.

Ferner kann eine erfindungsgemäße Zusammensetzung f) einen oder mehrere konventionell eingesetzte Hilfsstoffe enthalten. Derartige Hilfsstoffe, wie z.B. lipophile Tenside, Glycerol oder Propylenglykol sind hinlänglich bekannt,

Darüber hinaus betrifft die Erfindung ein Verfahren zur Herstellung einer Suspensionsdosieraerosolzusammensetzung, bei dem ein Gemisch das a) mindestens einen pharmazeutischen Wirkstoff, c) mindestens ein natives oder ein modifiziertes Cyclodextrin, d) mindestens ein hydrophiles Additiv umfaßt, das einen Polyethylenglykolanteil von 2 oder mehr Ethylenoxideinheiten besitzt, und gegebenenfalls e) Ethanol umfaßt, vorgelegt wird und das Gemisch durch Zugabe von b) mindestens einem Treibmittel, das aus 2H-Heptafluorpropan (HFA 227), 1,1,1,2-Tetrafluorethan (HFA 134a) oder einer Mischung aus beiden ausgewählt ist, in eine stabile, nicht-sedimentierende Suspension überführt wird.

Beispielsweise wird der pharmazeutische Wirkstoff zunächst durch Mischen mit Ethanol, Cyclodextrin und Polyethylenglykol bei Raumtemperatur in eine reale Lösung überführt. Gibt man zu dieser Lösung das vorher druckverflüssigte Treibmittel (HFA 227 oder 134a oder eine Mischung der beiden Treibmittel), so bildet sich ohne den Einfluß, äußerer Energie (Rühren, Dispergieren, Homogenisieren) spontan eine erfindungsgemäß milchige Suspension.

Auch das Dispergieren der Hilfsstoffe in einer Lösung des pharmazeutischen Wirkstoffes in Ethanol zu einer feinen Dispersion führt nach Zugabe eines druckverflüssigten Treibmittels spontan zu einer feinteiligen Suspension, die durch die vorhandenen Hilfsstoffe stabilisiert wird.

Die Vorteile der Erfindung werden durch die folgenden Beispiele verdeutlicht:

### Beispiel 1

36 mg Salbutamol werden mit 300 mg Polyethylenglykol 200, 600 mg Ethanol und 500 mg Hydroxypropyl-β-Cyclodextrin bei Raumtemperatur gelöst. Diese Lösung wird in eine druckresistente Glasflasche abgefüllt und mit einem Dosierventil, welches 50 µl pro Hub freisetzt, verschlossen. Durch das Ventil wird mit Treibmittel HFA 227 auf 12,5 g aufgefüllt. Pro Hub werden somit ca. 200 µg Salbutamol abgegeben.

### Beispiel 2

36 mg Salbutamol werden mit 250 mg Polyethylenglykol 300, 400 mg Ethanol und 500 mg Hydroxymethyl-β-Cyclodextrin bei Raumtemperatur gelöst. Diese Lösung wird in eine druckresistente Glasflasche abgefüllt und mit einem Dosierventil, welches 50 µl pro Hub freisetzt, verschlossen. Durch das Ventil wird mit Treibmittel HFA 227 auf 12,5 g aufgefüllt. Pro Hub werden somit ca. 200 µg Salbutamol abgegeben.

### Beispiel 3

36 mg Budesonid werden mit 200 mg Polyethylenglykol 300, 400 mg Ethanol und 160 mg Hydroxypropyl-β-Cyclodextrin bei Raumtemperatur gelöst. Diese Lösung wird in eine druckresistente Glasflasche abgefüllt und mit einem Dosierventil, welches 50 µl pro Hub freisetzt, verschlossen. Durch das Ventil wird mit Treibmittel HFA 227 auf 12,5 g aufgefüllt. Pro Hub werden somit ca. 200 µg Budesonid abgegeben.

### Beispiel 4

1,8 mg Formoterolfumaratdihydrat werden mit 200 mg Polyethylenglykol 600, 400 mg Ethanol und 100 mg Hydroxypropyl-β-Cyclodextrin bei Raumtemperatur gelöst. Diese Lösung wird in eine druckresistente Glasflasche abgefüllt und mit einem Dosierventil, welches 50 µl pro Hub freisetzt, verschlossen. Durch das Ventil wird mit Treibmittel HFA 227 auf 12,5 g aufgefüllt. Pro Hub werden somit ca. 10 µg Formoterolfumaratdihydrat abgegeben.

### Beispiel 5

36 mg Budesonid und 1,8 mg Formoterolfumaratdihydrat werden mit 200 mg Polyethylenglykol 300 , 400 mg Ethanol und 200 mg Hydroxypropyl-β-Cyclodextrin bei Raumtemperatur gelöst. Diese Lösung wird in eine druckresistente Glasflasche abgefüllt und mit einem Dosierventil, welches 50 µl pro Hub freisetzt, verschlossen. Durch das Ventil wird mit Treibmittel HFA 227 auf 12,5 g aufgefüllt. Pro Hub werden somit ca. 200 µg Budesonid und 10 µg Formoterolfumaratdihydrat abgegeben.

### Beispiel 6

45 mg Fluticason-17-propionat werden mit 300 mg Polyethylenglykol 300, 600 mg Ethanol und 300 mg Hydroxypropyl-β-Cyclodextrin bei Raumtemperatur gelöst. Diese Lösung wird in eine druckresistente Glasflasche abgefüllt und mit einem Dosierventil, welches 50 µl pro Hub freisetzt, verschlossen. Durch das Ventil wird mit Treibmittel HFA,227 auf 12,5 g aufgefüllt. Pro Hub werden somit ca. 250 µg Fluticason-17-propionat abgegeben.

### Beispiel 7

1,8 mg Formoterolfumaratdihydrat werden mit 200 mg Polyethylenglykol 600, 500 mg Ethanol und 20 mg Hydroxyethyl-β-Cyclodextrin bei Raumtemperatur gelöst. Diese Lösung wird in eine druckresistente Glasflasche abgefüllt und mit einem Dosierventil, welches 50 µl pro Hub freisetzt, verschlossen. Durch das Ventil wird mit Treibmittel HFA 227 auf 12,5 g aufgefüllt. Pro Hub werden somit ca. 10 µg Formoterölfumaratdihydrat abgegeben.

### Beispiel 8

9 mg Salmeterolxinafoat werden mit 300 mg Polyethylenglykol 300, 600 mg Ethanol und 40 mg Hydroxypropyl-β-Cyclodextrin bei Raumtemperatur gelöst. Diese Lösung wird in eine druckresistente Glasflasche abgefüllt und mit einem Dosierventil, welches 50 µl pro Hub freisetzt, verschlossen. Durch das Ventil wird mit Treibmittel HFA 227 auf 12,5 g aufgefüllt. Pro Hub werden somit ca. 50 µg Salmeterolxinafoat abgegeben.

### Beispiel 9

36 mg Budesonid werden mit 300 mg Polyethylenglykol 200, 600 mg Ethanol und 200 mg Hydroxypropyl-β-Cyclodextrin bei Raumtemperatur gelöst. Diese Lösung wird in eine druckresistente Glasflasche abgefüllt und mit einem Dosierventil, welches 50 µl pro Hub freisetzt, verschlossen. Durch das Ventil wird mit einer Mischung aus Treibmittel HFA 227 und HFA 134a im Verhältnis 80 zu 20 auf 12,5 g aufgefüllt. Pro Hub werden somit ca. 200 µg Budesonid abgegeben.

### Beispiel 10

10 mg Schweineinsulin werden mit 300 mg Polyethylenglykol 200, 600 mg Ethanol und 100 mg Hydroxypropyl-β-Cyclodextrin bei Raumtemperatur gelöst. Diese Lösung wird in eine druckresistente Glasflasche abgefüllt und mit einem Dosierventil, welches 50 µl pro Hub freisetzt, verschlossen. Durch das Ventil wird mit Treibmittel HFA 227 auf 12,5 g aufgefüllt. Pro Hub werden somit ca. 55 µg Schweineinsulin abgegeben.

## Patentansprüche

1. Suspensionsdosieraerosolzusammensetzung, die
a) mindestens einen pharmazeutischen Wirkstoff,
b) mindestens ein Treibmittel, das aus 2H-Heptafluorpropan (HFA 227), 1,1,1,2-Tetrafluorethan (HFA 134a) oder einer Mischung aus beiden ausgewählt ist,
c) mindestens ein natives oder ein modifiziertes Cyclodextrin und
d) mindestens ein hydrophiles Additiv umfaßt, das einen Polyethylenglykolanteil von 2 oder mehr Ethylenoxideinheiten besitzt,
wobei die Suspension dadurch hergestellt wird, daß ein Gemisch das a) mindestens einen pharmazeutischen- Wirkstoff, c) mindestens ein natives oder ein modifiziertes Cyclodextrin, d) mindestens ein hydrophiles Additiv umfaßt, das einen Polyethylenglykolanteil von 2 oder mehr Ethylenoxideinheiten besitzt, vorgelegt wird und das Gemisch durch Zugabe von b) mindestens einem Treibmittel, das aus 2H-Heptafluorpropan (HFA 227), 1,1,1,2-Tetrafluorethan (HFA 134a) oder einer Mischung aus beiden ausgewählt ist, in eine stabile, nicht-sedimentierende Suspension überführt wird.

2. Zusammensetzung nach Anspruch 1, **dadurch gekennzeichnet, daß** sie darüber hinaus e) Ethanol enthält.

3. Zusammensetzung nach einem der Ansprüche 1 oder 2, **dadurch gekennzeichnet, daß** der Wirkstoff
(i) ein Antiasthmatikum, insbesondere Budesonid, Beclometason, Dexamethason, Flunisolid, Fluticason, Hydrocortison, Triamcinolon, Adrenalin, Bitolterol, Clenbuterol, Ephedrin, Fenoterol, Formoterol, Isoproterenol, Noradrenalin, Pirbuterol, Reproterol, Salbutamol, Salmeterol, Terbutalin, Ipratropium, Oxitropium, Tiotropium, Nedocromil, Cromoglicinsäure oder Salze oder Ester der vorstehend genannten Verbindungen oder Kombinationen davon,
(ii) eine systemisch wirksame Substanz, insbesondere Atropin, Buprenorphin, Fentanyl, Morphin, Glibenclamid, Prednison, Prednisolon, Scopolamin, Sildenafil, Apomorphin oder deren Salze und Derivate sowie verwandte Substanzen,
(iii) ein Antiinfektivum, insbesondere Tobramycin, Gentamycin, Ciclosporin,
(iv) ein systemisch wirksames Protein, Peptid, Plasmid oder DNA-Fragment, insbesondere Insulin, α₁-Antitrypsin, Calcitonin, Dornase-α, Desmopressin, humanes Wachstumshormon und andere hormonell wirksame Substanzen,
(v) ein Prostaglandinderivat, insbesondere Alprosatdil, Prostaglandin E2 und andere Gewebshormone,
(vi) ein systemisch wirksames Vakzine oder Immunglobulin oder
(vii) eine hormonell wirksame Substanz ist.

4. Zusammensetzung nach einem der vorhergehenden Ansprüche, **dadurch gekennzeichnet, daß** das Cyclodextrin ein α-, β- oder γ-Cyclodextrin ist.

5. Zusammensetzung nach einem der vorhergehenden Ansprüche, **dadurch gekennzeichnet, daß** das modifizierte α-, β- oder γ-Cyclodextrin ein Hydroxymethyl-Cyclodextrin, Hydroxyethyl-Cyclodextrin, Hydroxypropyl-Cyclodextrin, Cyclodextrinbutylsulfonat, Cyclodextrinbutylfluorid und Sulfobutyl-Cyclodextrin ist.

6. Zusammensetzung nach einem der vorhergehenden Ansprüche, **dadurch gekennzeichnet, daß** der Gesamtgehalt der Komponenten c) und d) sowie gegebenenfalls e) 0,01 bis 30 Gew.-% beträgt, bevorzugt 0,1 bis 20 Gew.-%, insbesondere 0,5 bis 15 Gew.-%, bezogen auf die Gesamtmasse der Zubereitung.

7. Zusammensetzung nach einem der vorhergehenden Ansprüche, **dadurch gekennzeichnet, daß** das Gewichtsverhältnis Komponente a) Wirkstoff(e) zu Komponente c) Cyclodextrin(e) im Bereich von 10 zu 1 bis 1 zu 100, bevorzugt 5 zu 1 bis 1 zu 50, insbesondere 2 zu 1 bis 1 zu 20 liegt.

8. Zusammensetzung nach einem der vorhergehenden Ansprüche, **dadurch gekennzeichnet, daß** die Treibmittelkomponente b) 50 bis 99 Gew.-% ausmacht, bevorzugt 75 bis 98 Gew.-%, insbesondere 80 bis 97 Gew.-%, zum Beispiel 90 bis 95 Gew.-%, bezogen auf die Gesamtmasse der Zubereitung.

9. Zusammensetzung nach einem der vorhergehenden Ansprüche, **dadurch gekennzeichnet, daß** das hydrophile Additiv aus der Gruppe der Polyethylenglykole mit einem mittleren Molekulargewicht von 100 bis 3.0000, vorzugsweise mit einem mittleren Molekulargewicht von 200 bis 6000, und bevorzugt mit einem Molekulargewicht von 200 bis 1000, ausgewählt ist.

10. Zusammensetzung nach einem der vorhergehenden Ansprüche, **dadurch gekennzeichnet, daß** das hydrophile Additiv eine Mischung aus Polyethylenglykolen mit niedrigem und hohem Molekulargewicht darstellt.

11. Zusammensetzung nach einem der vorhergehenden Ansprüche, **dadurch gekennzeichnet, daß** das hydrophile Additiv aus der Gruppe der Polyethylenglykol-Fettsäureether (Brij-Substanzen), der Gruppe der Polyethylenglykol-Fettsäureester (Myrj-Substanzen), der Gruppe der Polyethylenglykol-Glycerol-Fettsäureester (z.B. Taga® S, Tagat® O, Solutol® HS 15, Cremophor® EL, Cremphor® S9), der Gruppe der Polysorbate oder der Gruppe der Polyoxyethylen-polypropylen-Block-CoPolymeren, z.B. Poloxamer® 188, Synperonic® P/E L 61, Pluronic® F 68, ausgewählt ist.

12. Zusammensetzung nach einem der vorhergehenden Ansprüche, **dadurch gekennzeichnet, daß** das hydrophile Additiv ein hydrophiles Polymer, insbesondere Polyvinylpyrrolidon, Polyvinylalkohol, quervernetztes Polyvinylpyrrolidon, Vinylpyrrolidon-Vinylacetat-Copolymer ist.

13. Verfahren zur Herstellung einer Suspensionsdosieraerosolzusammensetzung, bei dem ein Gemisch das a) mindestens einen pharmazeutischen Wirkstoff, c) mindestens ein natives oder ein modifiziertes Cyclodextrin, d) mindestens ein hydrophiles Additiv umfaßt, das einen Polyethylenglykolanteil von 2 oder mehr Ethylenoxideinheiten besitzt, und gegebenenfalls e) Ethanol umfaßt, vorgelegt wird und das Gemisch durch Zugabe von b) mindestens einem Treibmittel, das aus 2H-Heptafluorpropan (HFA 227), 1,1,1,2-Tetrafluorethan (HFA 134a) oder einer Mischung aus beiden ausgewählt ist, in eine stabile, nicht-sedimentierende Suspension überführt wird.

14. Verwendung einer Kombination mindestens eines nativen oder eines modifizierten Cyclodextrins (c) mit einem hydrophilen Additiv (d), das einen Polyethylenglykolanteil von 2 oder mehr Ethylenoxideinheiten besitzt, als Suspensionsstabilisator in einem mindestens ein Treibmittel (b), das aus 2H-Heptafluorpropan (HFA 227), 1,1,1,2-Tetrafluorethan (HFA 134a) oder einer Mischung aus beiden ausgewählt ist, und mindestens einen pharmazeutischen Wirkstoff (a) enthaltendenden Suspensionsdosieraerosol, wobei das Gemisch aus (a), (c), (d) und gegebenenfalls (e) vorgelegt wird und durch Zugabe von (b) eine stabile, nicht-sedimentierende Suspension gebildet wird.

## Claims

1. Suspension metered dose aerosol inhaler composition, which comprises
a) at least one pharmaceutical active ingredient,
b) at least one propellant that is selected from 2H-heptafluoropropane (HFA 227), 1,1,1,2-tetrafluoroethane (HFA 134a) or a mixture of both,
c) at least one natural or a modified cyclodextrin and
d) at least one hydrophilic additive that possesses a polyethylene glycol fraction of 2 or more ethylene oxide units,
wherein the suspension is manufactured such that a mixture is provided that comprises a) at least one pharmaceutical active ingredient, c) at least one natural or a modified cyclodextrin, d) at least one hydrophilic additive that possesses a polyethylene glycol fraction of 2 or more ethylene oxide units, and the mixture is converted into a stable, non-sedimenting suspension by the addition of b) at least one propellant that is selected from 2H-heptafluoropropane (HFA 227), 1,1,1,2-tetrafluoroethane (HFA 134a) or a mixture of both.

2. Composition according to Claim 1, **characterised in that** it additionally comprises e) ethanol.

3. Composition according to one of Claims 1 or 2, **characterised in that** the active ingredient is
(i) an anti-asthmatic agent, in particular budesonide, beclomethasone, dexamethasone, flunisolide, fluticasone, hydrocortisone, triamcinolone, adrenaline, bitolterol, clenbuterol, ephedrine, fenoterol, formoterol, isoproterenol, noradrenaline, pirbuterol, reproterol, salbutamol, salmeterol, terbutaline, ipratropium, oxitropium, tiotropium, nedocromil, cromoglycic acid or salts or esters of the abovementioned compounds or combinations thereof,
(ii) a systemically active substance, in particular atropine, buprenorphine, fentanyl, morphine, glibenclamide, prednisone, prednisolone, scopolamine, sildenafil, apomorphine or their salts and derivatives as well as related substances,
(iii) an anti-infective agent, in particular Tobramycin, Gentamycin, Ciclosporin,
(iv) a systemically active protein, peptide, plasmid or DNA fragment, in particular insulin, α₁-antitrypsin, calcitonin, dornase-α, desmopressin, human growth hormone and other hormonally active substances,
(v) a prostaglandin derivative, in particular Alprosatdil, Prostaglandin E2 and other tissue hormones,
(vi) a systemically active vaccine or immunoglobin or
(vii) a hormonally active substance.

4. Composition according to one of the preceding Claims, **characterised in that** the cyclodextrin is an α-, β- or γ-cyclodextrin.

5. Composition according to one of the preceding Claims, **characterised in that** the modified α-, β- or γ-cyclodextrin is a hydroxymethyl cyclodextrin, hydroxyethyl cyclodextrin, hydroxypropyl cyclodextrin, cyclodextrin butyl sulphonate, cyclodextrin butyl fluoride and sulphobutyl cyclodextrin.

6. Composition according to one of the preceding Claims, **characterised in that** the total content of the components c) and d) and optionally e) amounts to 0.01 to 30 wt.%, preferably 0.1 to 20 wt.%, in particular 0.5 to 15 wt.%, relative to the total weight of the preparation.

7. Composition according to one of the preceding Claims, **characterised in that** the weight ratio of component a) active ingredient(s) to component c) cyclodextrin(s) lies within the range of 10: 1 to 1: 100, preferably 5: 1 to 1: 50, in particular 2: 1 to 1: 20.

8. Composition according to one of the preceding Claims, **characterised in that** the propellant component b) amounts to 50 to 99 wt.%, preferably 75 to 98 wt.%, in particular 80 to 97 wt.%, for example 90 to 95 wt.%, relative to the total weight of the preparation.

9. Composition according to one of the preceding Claims, **characterised in that** the hydrophilic additive is selected from the group of polyethylene glycols with an average molecular weight of 100 to 30 000, advantageously with an average molecular weight of 200 to 6000 and preferably with a molecular weight of 200 to 1000.

10. Composition according to one of the preceding Claims, **characterised in that** the hydrophilic additive is a mixture of polyethylene glycols with low and high molecular weight.

11. Composition according to one of the preceding Claims, **characterised in that** the hydrophilic additive is selected from the group of polyethylene glycol fatty acid ethers (Brij substances), the group of polyethylene glycol fatty acid esters (Myrj substances), the group of polyethylene glycol glycerol fatty acid esters (e.g. Tagat® S, Tagat® O Solutol® HS 15, Cremophor® EL, Cremophor® S9), the group of polysorbates or the group of polyoxyethylene-polypropylene block copolymers, e.g. Poloxamer® 188, Synperonic® P/E L 61, Pluronic® F 68.

12. Composition according to one of the preceding Claims, **characterised in that** the hydrophilic additive is a hydrophilic polymer, in particular polyvinyl pyrrolidone, polyvinyl alcohol, cross-linked polyvinyl pyrrolidone, vinyl pyrrolidone-vinyl acetate copolymer.

13. Process for manufacturing a suspension metered dose aerosol inhaler composition, in which a mixture is provided that comprises a) at least one pharmaceutical active ingredient, c) at least one natural or a modified cyclodextrin, d) at least one hydrophilic additive that possesses a polyethylene glycol fraction of 2 or more ethylene oxide units, and the mixture is converted into a stable, non-sedimenting suspension by the addition of b) at least one propellant that is selected from 2H-heptafluoropropane (HFA 227), 1,1,1,2-tetrafluoroethane (HFA 134a) or a mixture of both.

14. Use of a combination of at least one natural or a modified cyclodextrin (c) with at least one hydrophilic additive (d) that possesses a polyethylene glycol fraction of 2 or more ethylene oxide units as the suspension stabiliser in a suspension metered dose aerosol inhaler comprising at least one propellant (b) that is selected from 2H-heptafluoropropane (HFA 227), 1,1,1,2-tetrafluoroethane (HFA 134a) or a mixture of both, and at least one pharmaceutical active ingredient (a), wherein the mixture of (a), (c), (d) and optionally (e) is present and a stable, non-sedimenting suspension is formed by adding (b).

## Revendications

1. Composition pour aérosol doseur de suspension, comprenant
a) au moins une substance active pharmaceutique,
b) au moins un propulseur choisi parmi le 2H-heptafluoropropane (HFA 227), le 1,1,1,2-tétrafluoroéthane (HFA 134a) ou un mélange des deux,
c) au moins une cyclodextrine native ou modifiée et
d) au moins un additif hydrophile ayant une teneur en polyéthylèneglycol de 2 ou plus de deux motifs oxyde d'éthylène,
la suspension étant préparée par disposition au préalable d'un mélange comprenant a) au moins une substance active pharmaceutique, c) au moins une cyclodextrine native ou modifiée, d) au moins un additif hydrophile ayant une teneur en polyéthylèneglycol de 2 ou plus de deux motifs oxyde d'éthylène, et conversion du mélange en une suspension stable, ne se sédimentant pas, par addition de b) au moins un propulseur choisi parmi le 2H-heptafluoropropane (HFA 227), le 1,1,1,2-tétrafluoroéthane (HFA 134a) ou un mélange des deux.

2. Composition selon la revendication 1, **caractérisée en ce qu'**elle contient en outre e) de l'éthanol.

3. Composition selon la revendication 1 ou 2, **caractérisé en ce que** la substance active est
(i) un antiasthmatique, en particulier le budésonide, la béclométasone, la dexaméthasone, le flunisolide, la fluticasone, l'hydrocortisone, la triamcinolone, l'adrénaline, le bitoltérol, le clenbutérol, l'éphédrine, le fénotérol, le formotérol, l'isoprotérénol, la noradrénaline, le pirbutérol, le réprotérol, la salbutamol, le salmétérol, la terbutaline, l'ipratropium, l'oxitropium, le tiotropium, le nédocromil, l'acide cromoglicique ou des sels ou esters des composés précités ou des associations de ceux-ci,
(ii) une substance à action systémique, en particulier l'atropine, la buprénorphine, le fentanyl, la morphine, le glibenclamide, la prednisone, la prednisolone, la scopolamine, le sildénafil, l'apomorphine ou leurs sels ou dérivés ainsi que des substances apparentées,
(iii) un agent anti-infectieux, en particulier la tobramycine, la gentamicine, la cyclosporine,
(iv) une protéine, un peptide, un plasmide ou un fragment d'ADN, à action systémique, en particulier l'insuline, l'α₁-antitrypsine, la calcitonine, la dornase-α, la desmopressine, l'hormone de croissance humaine et d'autres substances à activité hormonale,
(v) un dérivé de prostaglandine, en particulier l'alprosatdil, la prostaglandine E2 et d'autres hormones tissulaires,
(vi) un vaccin ou une immunoglobuline à action systémique ou
(vii) une substance à activité hormonale.

4. Composition selon l'une quelconque des revendications précédentes, **caractérisée en ce que** la cyclodextrine est une α-, β- ou γ-cyclodextrine.

5. Composition selon l'une quelconque des revendications précédentes, **caractérisée en ce que** l'α-, β- ou γ-cyclodextrine est une hydroxyméthyl-cyclodextrine, hydroxyéthyl-cyclodextrine, hydroxypropyl-cyclodextrine, cyclodextrine-butylsulfonate, cyclodextrine-butylfluorure ou sulfobutylcyclodextrine.

6. Composition selon l'une quelconque des revendications précédentes, **caractérisée en ce que** la teneur totale en les composants c) et d) ainsi qu'éventuellement e) va de 0,01 à 30 % en poids, de préférence de 0,1 à 20 % en poids, en particulier de 0,5 à 15 % en poids, par rapport à la masse totale de la préparation.

7. Composition selon l'une quelconque des revendications précédentes, **caractérisée en ce que** le rapport pondéral du composant a) substance(s) active(s) au composant c) cyclodextrine(s) se situe dans la plage allant de 10:1 à 1:100, de préférence de 5:1 à 1:50, en particulier de 2:1 à 1:20.

8. Composition selon l'une quelconque des revendications précédentes, **caractérisée en ce que** le composant propulseur b) représente de 50 à 99 % en poids, de préférence de 75 à 98 % en poids, en particulier de 80 à 97 % en poids, par exemple de 90 à 95 % en poids, par rapport à la masse totale de la préparation.

9. Composition selon l'une quelconque des revendications précédentes, **caractérisée en ce que** l'additif hydrophile est choisi dans le groupe des polyéthylèneglycols ayant une masse moléculaire moyenne de 100 à 30 000, de préférence ayant une masse moléculaire moyenne de 200 à 6 000, préférentiellement ayant une masse moléculaire de 200 à 1 000.

10. Composition selon l'une quelconque des revendications précédentes, **caractérisée en ce que** l'additif hydrophile représente un mélange de polyéthylèneglycols de faible masse moléculaire et de polyéthylèneglycols de masse moléculaire élevée.

11. Composition selon l'une quelconque des revendications précédentes, **caractérisée en ce que** l'additif hydrophile est choisi dans le groupe des éthers d'acides gras et de polyéthylèneglycol (substances Brij), le groupe des esters d'acides gras et de polyéthylèneglycol (substances Myrj), le groupe des esters d'acides gras et de glycérolpolyéthylèneglycol (par exemple Tagat® S, Tagat® O, Solutol® HS 15, Cremophor® EL, Cremophor® S9), le groupe des Polysorbate ou le groupe des copolymères en blocs polyoxyéthylène-polyoxypropylène, par exemple Poloxamer® 188, Synperonic® P/EL 61, Pluronic® F 68.

12. Composition selon l'une quelconque des revendications précédentes, **caractérisée en ce que** l'additif hydrophile est un polymère hydrophile, en particulier la polyvinylpyrrolidone, le poly(alcool vinylique), la polyvinylpyrrolidone réticulée, un copolymère vinylpyrrolidone/acétate de vinyle.

13. Procédé pour la préparation d'une composition pour aérosol doseur de suspension, dans lequel on dispose au préalable un mélange comprenant a) au moins une substance active pharmaceutique, c) au moins une cyclodextrine native ou modifiée, d) au moins un additif hydrophile ayant une teneur en polyéthylèneglycol de 2 ou plus de deux motifs oxyde d'éthylène et le cas échéant e) de l'éthanol, et on convertit le mélange en une suspension stable, ne se sédimentant pas, par addition de b) au moins un propulseur choisi parmi le 2H-heptafluoropropane (HFA 227), le 1,1,1,2-tétrafluoroéthane (HFA 134a) ou un mélange des deux.

14. Utilisation d'une association d'au moins une cyclodextrine native ou modifiée (c) avec un additif hydrophile (d) ayant une teneur en polyéthylèneglycol de 2 ou plus de deux motifs oxyde d'éthylène, en tant que stabilisant de suspension dans un aérosol doseur de suspension contenant au moins un propulseur (b) choisi parmi le 2H-heptafluoropropane (HFA 227), le 1,1,1,2-tétrafluoroéthane (HFA 134a) ou un mélange des deux, et au moins une substance active pharmaceutique (a) ; le mélange de (a), (c), (d) et éventuellement (e) étant disposé au préalable et une suspension stable, ne se sédimentant pas, étant formée par addition de (b).
